Europäisches Patentamt

European Patent Office

Office européen des brevets

(11) Veröffentlichungsnummer: **0 248 358 B1**

(12) **EUROPÄISCHE PATENTSCHRIFT**

(45) Veröffentlichungstag der Patentschrift: **02.09.92**

(21) Anmeldenummer: **87107794.7**

(22) Anmeldetag: **29.05.87**

(51) Int. Cl.5: **C07C 231/00**, C07C 233/58

(54) Verfahren zur Verringerung des Kupfersalzgehalts in Oxamid.

(30) Priorität: **04.06.86 DE 3618816**

(43) Veröffentlichungstag der Anmeldung:
**09.12.87 Patentblatt 87/50**

(45) Bekanntmachung des Hinweises auf die Patenterteilung:
**02.09.92 Patentblatt 92/36**

(84) Benannte Vertragsstaaten:
**AT DE ES FR GB IT NL**

(56) Entgegenhaltungen:
**EP-A- 0 245 632**
**EP-A- 0 271 161**
**CH-A- 348 399**

(73) Patentinhaber: **HOECHST AKTIENGESELL-SCHAFT**
**Postfach 80 03 20**
**W-6230 Frankfurt am Main 80(DE)**

(72) Erfinder: **Riemenschneider, Wilhelm, Dr.**
**Loreleistrasse 45**
**W-6230 Frankfurt am Main 80(DE)**

**Beschreibung**

Die vorliegende Erfindung betrifft ein Verfahren zum Verringern des Kupfergehalts in Oxamid, welches unter Verwendung eines kupfersalzhaltigen Katalysators hergestellt wurde.

Oxalsäurediamid, kurz Oxamid genannt, hat als Düngemittel mit Langzeitwirkung industrielle Bedeutung erlangt. Außerdem kann Oxamid als Zwischenprodukt für organische Synthesen, z.B. für Umsetzungen mit Formaldehyd, Essigsäure, Hydrazin und ähnlichem verwendet werden.

Es sind für Oxamid eine Reihe von Synthesen beschrieben, die in den meisten Fällen von Dicyan oder Cyanwasserstoff ausgehen. Besonders die Synthese des Oxamids aus Cyanwasserstoff, Sauerstoff und Wasser in einem Einstufen-Prozeß - s. Chemical Eng. May 1976, Seite 69-70 und DE-PS 23 08 941 (1975) - hat technische Bedeutung erlangt.

Die genannten Oxamid-Synthesen arbeiten mit kupferhaltigen Katalysatoren. Diese Arbeitsweise hat den Nachteil, daß Oxamid als festes Endprodukt von der Synthese her noch eine gewisse Menge Kupfer enthält. Da auf die katalytische Wirkung des Kupfers nicht verzichtet werden kann, ist ein gewisser Kupfergehalt des rohen Endproduktes (technisches Oxamid) nicht vermeidbar. Im allgemeinen enthält das nach DE-PS 23 08 941 hergestellte Oxamid je nach Konzentration der verwendeten Katalysatorlösung 500 bis 1500 ppm Kupfer. Arbeitet man unter wirtschaftlich optimalen Reaktionsbedingungen, so enthält das entstehende Oxamid ca. 600-1350 ppm Kupfer. Dieser Wert ist für die meisten Verwendungszwecke des Oxamids zu hoch.

Für die technische Anwendung, z.B. für die Verwendung als Düngemittel, ist eine vollständige Eliminierung des Kupfers nicht notwendig, sondern eine Verringerung des Kupfergehalts von ca. 1000 ppm auf Werte zwischen 500 und 100 ppm durchaus ausreichend. Der Kupfergehalt des synthetisierten Oxamids von ca. 1000 ppm ist aber durch einfaches Waschen, selbst mit sehr viel Wasser oder mit verdünnten Säuren oder Laugen oder anderen Lösungsmitteln nicht oder nur unwesentlich absenkbar.

Es bestand also die Aufgabe, den Kupfergehalt des auf oben genannten Wegen hergestellten Oxamids auf einen wesentlich kleineren und tolerierbaren Wert zu verringern.

Eine weitere Aufgabe ist es, die notwendige Reinigungsoperation möglichst kostenarm durchzuführen, weil insbesondere bei der Verwendung als Düngemittel nur geringe Unkosten noch wirtschaftlich tragbar sind.

In der EP-A 0245632 wird ein Verfahren zur Entfernung von Kupfer aus Oxamid, das unter Verwendung eines kupfersalzhaltigen Katalysators hergestellt wurde, vorgeschlagen. Das Verfahren ist dadurch gekennzeichnet, daß Komplexbildner eingesetzt werden, die mit Kupfer wasserlösliche Komplexe bilden und einen pH-Wert in der Lösung verursachen, der zwischen 2 und 8 liegt.

Es ist ferner bekannt (s. Gmelins Handbuch d. Anorganischen Chemie, 8. Auflage (1966, Kupfer Teil B, Lieferung 4, System-Nr. 60, Seite 1661), daß man z. B. Nitrilotriessigsäure zum "Entkupfern" von Garnen und Geweben verwenden kann.

Die vorliegende Erfindung betrifft ein Verfahren zur Verringerung des Kupfergehalts in Oxamid, das Kupfer enthält, wobei eine Suspension bestehend aus kupferhaltigem Oxamid, mindestens einem Komplexierungsmittel und Wasser bei einer Temperatur von 20 bis 100°C verrührt wird und das kupferarme Oxamid anschließend von der Lösung abgetrennt wird, dadurch gekennzeichnet, daß als Komplexierungsmittel Ammoniak verwendet wird.

Das Abtrennen von der Lösung erfolgt durch bekannte Maßnahmen, wie Dekantieren, Zentrifugieren oder Absaugen. Das Verfahren läßt sich technisch einfach und mit geringem Aufwand durchführen.

Es war nicht zu erwarten, daß ein einfaches Rühren in einer Supension mit Ammoniak als komplexbildende Substanz zu einer Reduzierung des Kupfergehalts auf Werte von 20 - 500 ppm im Oxamid führt. Das einfache Ausrühren mit Wasser allein, auch im sauren oder alkalischen Bereich, bleibt nahezu ohne Effekt. Erst ein Zusatz von $NH_3$ bringt die gewünschte Reduzierung des Kupfergehaltes.

Als besonders geeignet hat sich das Ausrühren mit wäßrigem Ammoniak erwiesen, wobei die Konzentration der wäßrigen Lösung nicht unter 0,5n und vorzugsweise nicht unter 1n entsprechend 1,7 Gew.-% $NH_3$ liegen sollte. Nach oben ist der $NH_3$-Konzentration nur durch die Löslichkeit von Ammoniak in Wasser eine Grenze gesetzt; ein Arbeiten mit einer handelsüblichen technischen Ammoniaklösung von etwa 30% $NH_3$-Gehalt führt zu ausgezeichneten Ergebnissen. Die Konzentration der oben genannten anderen komplexbildenden Verbindungen sollte in ähnlichen Grenzen zwischen ca. 0,1 Gew.-% und 30 Gew.-% gehalten werden, soweit die Löslichkeit des Komplexbildners in Wasser das erlaubt.

Die Behandlung des kupferhaltigen Oxamids erfolgt vorzugsweise im einfachen Rührkessel. Die optimale Rührzeit ist von der Konzentration und von der Temperatur abhängig und liegt zwischen 0,1 und 10 Stunden, insbesondere 1-5 Stunden. So ist z.B. mit einer wäßrigen Lösung von 30 gewichtsprozentigem $NH_3$ eine Rührzeit von 1 Stunde bei Raumtemperatur ausreichend, während mit 1,7 gewichtsprozentigemNH₃ ca. 5 Stunden bei 80 bis 90°C gerührt werden sollte. Diese Zeitangaben sollen jedoch nur Anhaltswerte sein, die gegebenenfalls auch über- oder unterschritten werden können.

Die Menge der angewandten wäßrigen Lösung von NH₃ ist nicht kritisch, soll jedoch so bemessen werden, daß noch eine gut rührbare Suspension des Oxamids in der Flüssigkeit resultiert. Mögliche Zusammensetzungsbereiche Oxamid/Flüssigkeit sind z.B. 1:1 bis 1:100; besser jedoch 1:1 bis 1:75. Bevorzugt sind Aufschlämmungen von einem Teil Oxamid in 4 Teilen (= 20 %ige Suspension) bis zu 50 Teilen (= 2 %ige Suspension) der wäßrigen Ausrührlösung.

Normalerweise ist bei ausreichender Konzentration und ausreichender Zeit ein Verrühren bei Raumtemperatur ausreichend. Eine Erhöhung der Temperatur in Bereiche bis maximal 100°C beschleunigt jedoch die Kupferentfernung beträchtlich. So ist z.B. mit verdünnter wäßriger NH₃-Lösung beim Arbeiten bei 90°C die gewünschte Entkupferung schon in einem Fünftel der Zeit im Vergleich zur Raumtemperatur möglich. Am wirtschaftlichsten hat sich der Temperaturbereich von 20 - 50°C erwiesen.

Der Druck ist für den Erfolg des Verfahrens nicht kritisch, beispielsweise erzielt man bei einem Überdruck von 3 bar die gleichen Ergebnisse.

Das Oxamid wird nach Beendigung des Rührens mit bekannten Maßnahmen, wie z.B. Dekantieren, Zentrifugieren oder Abfiltrieren von der Flüssigphase abgetrennt, gewaschen und anschließend getrocknet. Das herausgelöste Kupfer befindet sich in der abgetrennten Flüssigkeit.

Auf diese Weise kann der Kupfergehalt des Oxamids von ca. 1000 ppm auf Werte zwischen 500 und 10 ppm gesenkt werden.

Man kann zwar kupferhaltiges Oxamid durch Umkristallisieren aus Wasser reinigen. Wegen seiner schlechten Löslichkeit sind hierfür große Mengen Wasser erforderlich. Dennoch wird durch dieses Verfahren der Kupfergehalt nicht entscheidend verringert (s. vgl. Beispiel 16).

Es ist daher überraschend, daß nach dem erfindungsgemäßen Verfahren ein Oxamid mit niedrigerem Kupfergehalt gewonnen werden kann.

Im folgenden wird die Erfindung durch Beispiele näher erläutert.

Beispiele:

Das bei allen Beispielen verwendete Oxamid stammt aus einer Herstellung nach DE-PS 23 08 941 und enthält 0,132 % = 1320 ppm Kupfer. Das Oxamid wird in allen Beispielen mit der Ausrührflüssigkeit verrührt, nach Abkühlen bei Raumtemperatur abgesaugt mit Wasser nachgewaschen und getrocknet.

| Beispiel | eingesetzte Oxamid-menge [g] | Ausrührflüssigkeit | | Arbeitsbedingungen | | | Cu-Gehalt d. Oxamids in ppm |
|---|---|---|---|---|---|---|---|
| | | Menge [g] | Zusammensetzung | Rührzeit [Std.] | Temp. [°C] | Ausbeute [%] | nach Behandl. |
| Vergl. Beisp. | 10 | 40 | reines Wasser | 1 | 90 | 99,5 | 1270 |
| 1 | " | " | 2 Gew.-% $NH_3$ in Wasser | 5 | 20 | 99,6 | 460 |
| 2 | " | " | 10 " " " | 4 | 20 | 99,4 | 112 |
| 3 | " | " | 30 " " " | 4 | 20 | 99,2 | 92 |
| 4 | " | 50 | 1,7 " " " | 1 | 90 | 98,8 | 183 |
| 5 | " | " | 1,7 " " " | 1 | 50 | 99,1 | 272 |
| 6 | " | " | 10 " " " | 2 | 50 | 98,6 | 30 |
| 7 | " | " | 20 " " " | 2 | 50 | 99,3 | 13 |
| 8 | " | 200 | 1,7 " " " | 1 | 50 | 98,3 | 160 |
| 9 | " | 500 | 1,7 " " " | 1 | 50 | 97,7 | 106 |

Beispiel 10 (Vergleichsbeispiel)

Oxamid von Beispiel 1 wurde durch Umkristallisieren aus kochendem Wasser gereinigt. Nach dieser Behandlung lag der Kupfergehalt bei ca. 500 ppm.

**Patentansprüche**

1. Verfahren zur Verringerung des Kupfergehalts in Oxamid, das Kupfer enthält, wobei eine Suspension bestehend aus kupferhaltigem Oxamid, mindestens einem Komplexierungsmittel und Wasser bei einer Temperatur von 20 bis 100°C verrührt wird und das kupferarme Oxamid anschließend von der Lösung abgetrennt wird, dadurch gekennzeichnet, daß als Komplexierungsmittel Ammoniak verwendet wird.

2. Verfahren nach Anspruch 1, dadurch gekennzeichnet, daß eine wäßrige Ammoniak-Lösung in einer

4

Konzentration von 1,7 - 30 Gew.-% eingesetzt wird.

3.  Verfahren nach einem der Ansprüche 1 oder 2, dadurch gekennzeichnet, daß das Mischungsverhältnis Oxamid/Wasser in der eingesetzten Suspension zwischen 1:1 und 1:100 liegt.

4.  Verfahren nach Anspruch 3, dadurch gekennzeichnet, daß das Mischungsverhältnis Oxamid/Wasser in der eingesetzten Suspension zwischen 1:4 und 1:50 liegt.

5.  Verfahren nach einem der Ansprüche 1 oder 2, dadurch gekennzeichnet, daß die Suspension nach dem Verrühren eine Temperatur von 80 - 100°C aufweist und vor dem Abtrennen des gereinigten Oxamids abgekühlt wird.

## Claims

1.  A process for reducing the copper content of oxamide containing copper, a suspension composed of copper-containing oxamide, at least one complexing agent and water being stirred at a temperature from 20 to 100°C and the oxamide of low copper content then being separated off from the solution, which comprises employing ammonia as complexing agent.

2.  The process as claimed in claim 1, wherein an aqueous ammonia solution of a concentration of 1.7-30% by weight is employed.

3.  The process as claimed in either of claims 1 or 2, wherein the oxamide/water mixing ratio in the suspension employed is between 1:1 and 1:100.

4.  The process as claimed in claim 3, wherein the oxamide/water mixing ratio in the suspension employed is between 1:4 and 1:50.

5.  The process as claimed in either of claims 1 or 2, wherein the suspension has a temperature of from 80° to 100° after stirring and is cooled before the purified oxamide is separated off.

## Revendications

1.  Procédé pour réduire la teneur en cuivre dans un oxamide renfermant du cuivre, au cours duquel on agite une suspension constituée d'oxamide renfermant du cuivre, d'au moins un agent complexant et d'eau, à une température comprise entre 20 et 100°C, et on sépare ensuite l'oxamide appauvri en cuivre de la solution, caractérisé en ce qu'on utilise l'ammoniac comme agent complexant.

2.  Procédé selon la revendication 1, caractérisé en ce qu'on utilise une solution aqueuse d'ammoniac à une concentration de 1,7 à 30% en poids.

3.  Procédé selon l'une des revendications 1 ou 2, caractérisé en ce que le rapport de mélange oxamide/eau est compris entre 1:1 et 1:100 dans la suspension utilisée.

4.  Procédé selon la revendication 3, caractérisé en ce que le rapport de mélange oxamide/eau est compris entre 1:4 et 1:50 dans la suspension utilisée.

5.  Procédé selon l'une des revendications 1 ou 2, caractérisé en ce que la suspension présente une température de 80 A 100°C après agitation et on la refroidit avant de séparer l'oxamide purifié.